# EUROPEAN PATENT APPLICATION

(11) **EP 2 335 596 A1**
(43) Date of publication of application: **22.06.2011**
(21) Application number: 10194835.4
(22) Date of filing: 14.12.2010
(51) Int. Cl.: A61B 8/14, G01S 15/89

(54) **Ultrasound system and method of selecting slice image from three-dimensional ultrasound image**

(30) Priority: 15.12.2009 KR 20090124913; 01.12.2010 KR 20100121158
(71) Applicant: MEDISON CO., LTD., Kangwon-do 250-870 (KR)
(72) Inventor: Yoo, Bong Soo, 135-851, Seoul (KR); Choi, Young Min, 135-851, Seoul (KR)
(74) Representative: Schmid, Wolfgang

(57) **Abstract**

Various embodiments of ultrasound system and method of selecting a 2D slice image from a 3D ultrasound image are provided. One embodiment of the ultrasound system comprises: a 3D ultrasound image acquisition unit configured to acquire a 3D ultrasound image of a target object; a 2D slice image selection unit including a control volume unit, the control volume unit being configured to be rotated and/or moved by an operator; and a processor coupled to the 3D ultrasound image acquisition unit and the 2D slice image selection unit. At least one selection plane is formed by the control volume unit as a reference plane to select at least one 2D slice image from the 3D ultrasound image. The processor is configured to extract at least one 2D slice image corresponding to the at least one selection plane from the 3D ultrasound image. The 3D ultrasound image or the at least one selection plane are rotated and/or moved together with the control volume unit.

## Description

### CROSS-REFERENCE TO RELATED APPLICATIONS

This application claims priority to Korean Patent Application No. 10-2009-0124913 filed on December 15, 2009 and Korean Patent Application No. 10-2010-0121158 filed on December 01, 2010.

### BACKGROUND

The present disclosure generally relates to ultrasound systems, and more particularly to an ultrasound system having an apparatus for selecting a slice image of a three-dimensional ultrasound image through a control volume unit and a method of selecting a two-dimensional slice image of the three-dimensional ultrasound image.

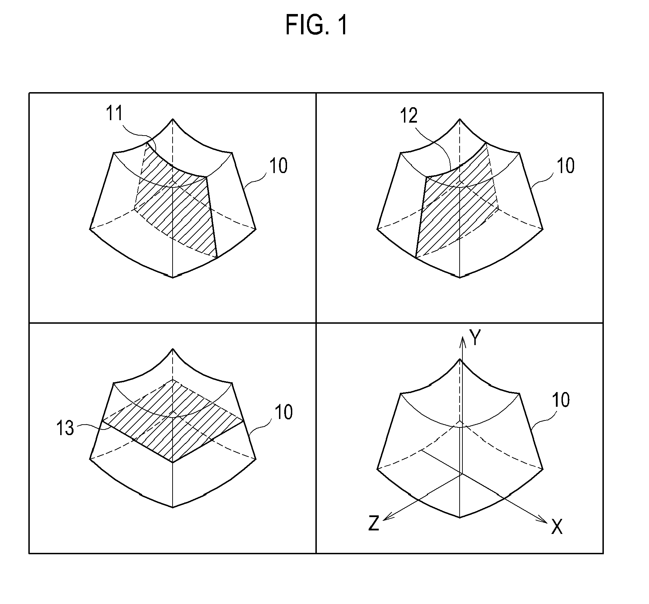

Three-dimensional ultrasound probes acquires three-dimensional volume images (hereinafter referred to as 3D ultrasound images) by steering and triggering transducer elements to emit ultrasound signals and receiving echo ultrasound signals reflected from a target object. Such 3D ultrasound image can be changed in its representation according to a steering way of the transducer elements in the 3D ultrasound probe. Referring to FIG. 1, there is shown an exemplary 3D ultrasound image 10 acquired through steered transducer elements of a conventional ultrasound probe. As shown in FIG. 1, the 3D ultrasound image 10 obtained by the conventional ultrasound probe corresponds to transmitting/receiving regions of the ultrasound signal. Thus, an upper portion of the 3D ultrasound image 10 is represented in a shape similar to a curved shape of a rectangular tracing a steered path of the transducer elements so that the 3D ultrasound image 10 can be a trapezoid (e.g., frustum) having a lower portion wider than the upper portion.

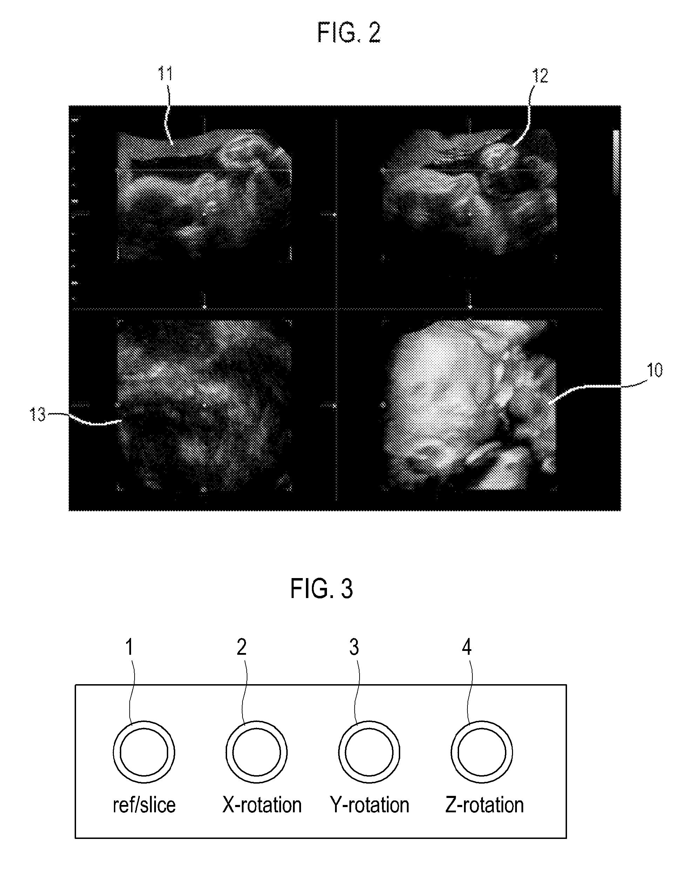

A cross-section of the 3D ultrasound image 10 can be obtained in the form of a two-dimensional (2D) ultrasound image (i.e., 2D slice image) at a region of interest (ROI) of a target object (not shown), which can be selected by an operator. FIG. 1 shows a Z-axis reference plane 11, an X-axis reference plane 12 and a Y-axis reference plane 13. Referring to FIG. 2, there are shown 2D slice images obtained on the respective reference planes 11, 12 and 13 and a 3D ultrasound image. To display a 2D slice image at an ROI of the 3D ultrasound image, the reference planes 11, 12 and 13 should be moved to coordinates corresponding to the ROI. For example, the operator can select the reference planes 11, 12 and 13 to move and rotate the selected planes to obtain a 2D slice image at the ROI of the 3D ultrasound image. The operator can perform a diagnosis for the target object through the obtained 2D slice image.

Referring to FIG. 3, there is shown a schematic diagram of a control panel to select a 2D slice image at an ROI of the 3D ultrasound image. As shown in FIG. 3, the control panel includes a ref/slice button 1, an X-rotation button 2, a Y-rotation button 3 and a Z-rotation button 4. The ref/slice button 1 is configured to select one of the reference planes 11, 12 and 13 and move the selected one in its corresponding axis direction. The X-rotation button 2 is configured to rotate the selected reference plane centering on an X-axis. The Y-rotation button 3 is configured to rotate the selected reference plane centering on a Y-axis. The Z-rotation button 4 is configured to rotate the selected reference plane centering on a Z-axis.

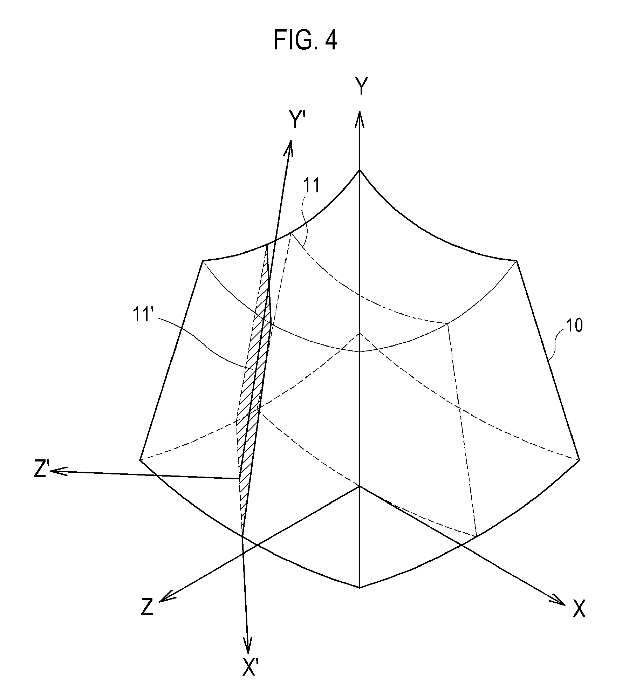

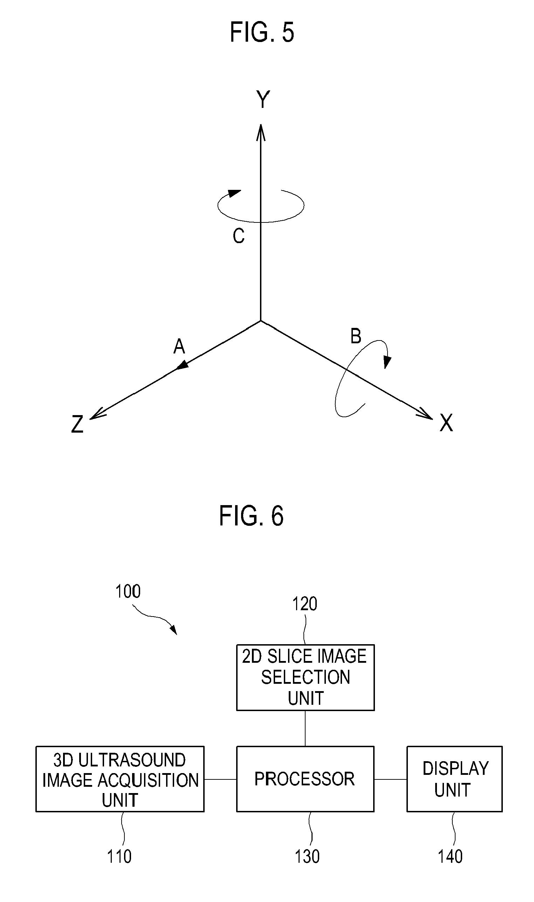

For example, selecting a 2D slice image 11' shown in FIG. 4 can be accomplished through the following procedure. First, the operator moves the reference plane 11 on the Z-axis toward an arrow A, as shown in FIG. 5, by using the ref/slice button 1. Then, the operator rotates the reference plane 11 centering on the X-axis toward an arrow B, as shown in FIG. 5, by using the X-rotation button 2. Lastly, the operator rotates the reference plane 11 centering on the Y-axis toward an arrow C, as shown in FIG. 5, by using the Y-rotation button 3. Thereafter, it becomes possible to obtain the selected 2D slice image 11' transited from the reference plane 11 according to the aforementioned procedure. Such operations described above, however, require a plurality of key manipulations, which is time-consuming and becomes complicated tasks to the operator. Moreover, it is difficult to obtain a precise 2D slice image, i.e., a slice image, from the 3D ultrasound image through the aforementioned manual operation.

### SUMMARY

Various embodiments of an ultrasound system having an apparatus for selecting a slice image of a 3D ultrasound image and a method of selecting a 2D slice image from a 3D ultrasound image are provided. In one embodiment of the present disclosure, by way of non-limiting example, the ultrasound system comprises: a 3D ultrasound image acquisition unit configured to acquire a 3D ultrasound image of a target object; a 2D slice image selection unit including a control volume unit, the 2D slice image selection unit being configured to be rotated and/or moved by an operator; and a processor coupled to the 3D ultrasound image acquisition unit and 2D slice image selection unit. At least one selection plane is formed by the control volume unit as a reference plane for selecting at least one 2D slice image from the 3D ultrasound image. The processor is configured to extract at least one 2D slice image corresponding to the at least one selection plane from the 3D ultrasound image. The 3D ultrasound image or the at least one selection plane are rotated and/or moved together with the control volume unit.

The at least one selection plane is fixed while the control volume unit and the 3D ultrasound image are rotated and/or moved together. Further, the processor matches a coordinate system of the control volume unit to a coordinate system of the 3D ultrasound image to rotate and/or move the 3D ultrasound image and the control volume unit together relative to the at least one selection plane. Alternatively, the 3D ultrasound image is fixed while the control volume unit and the at least one selection plane are rotated and/or moved together, and the processor matches a coordinate system of the control volume unit to a coordinate system of the at least one selection plane to rotate and/or move the at least one selection plane and the control volume unit together relative to the 3D ultrasound image.

The shape of the control volume unit corresponds to the shape of the 3D ultrasound image.

The 2D slice image selection unit includes: an orientation and position recognition unit mounted on the control volume unit; a grip coupled to the control volume unit; and an operation button formed on the grip. The orientation and position recognition unit is configured to detect the rotation and/or movement of the control volume unit to form an orientation and position signal of the control volume unit. The operation button is configured to receive input data for operations of the 2D slice image selection unit from the operator.

The orientation and position recognition unit includes a sensor that is configured to detect the rotation and/or movement of the control volume unit to form detection signals. Further, the processor is configured to generate the orientation and position signal of the control volume unit based on the detection signals.

The processor includes: a matching unit configured to match the coordinate system of the control volume unit to the coordinate system of the 3D ultrasound image; an image processing unit configured to change the orientation and position of the 3D ultrasound image or the at least one selection plane corresponding to the changed orientation and position of the control volume unit based on the orientation and position signal; and a 2D slice image extraction unit configured to extract at least one 2D slice image corresponding to the at least one selection plane from the 3D ultrasound image.

If the operation button receives input data of a first operation, then the matching unit matches the coordinate system of the control volume unit to the coordinate system of the 3D ultrasound image. If the operation button receives input data of a second operation, then the 2D slice image extraction unit extracts the at least one 2D slice image corresponding to the at least one selection plane from the 3D ultrasound image.

Further, in one embodiment of the present disclosure, the method of selecting a 2D slice image from a 3D ultrasound image comprises the following steps : a) acquiring a 3D ultrasound image of a target object; b) matching a coordinate system of the 3D ultrasound image to a coordinate system of a control volume unit configured to be moved and/or rotated by an operator, wherein at least one selection plane is formed by the control volume unit as a reference plane for selecting at least one 2D slice image from the 3D ultrasound image; c) detecting orientation and position of the control volume unit; d) rotating and/or moving the 3D ultrasound image or the at least one selection plane together with the control volume unit; and e) extracting at least one 2D slice image corresponding to the at least one selection plane from the 3D ultrasound image.

The at least one selection plane is fixed while the control volume unit and the 3D ultrasound image are rotated and/or moved together. Otherwise, the 3D ultrasound image is fixed while the control volume unit and the at least one selection plane are rotated and/or moved together.

### BRIEF DESCRIPTION OF THE DRAWINGS

- FIG. 1: is an exemplary a three-dimensional (3D) ultrasound image acquired through steered transducer elements of a conventional ultrasound probe.
- FIG. 2: is 2D slice images obtained on respective reference planes and a 3D ultrasound image by using the conventional ultrasound probe.
- FIG. 3: is a front view of a conventional control panel for selecting a 2D slice image from the 3D ultrasound image.
- FIG. 4: is a diagram for illustrating a selection process of a 2D slice image from the 3D ultrasound image.
- FIG. 5: is a diagram for illustrating a transition process of coordinates during the selection process shown in FIG. 4.
- FIG. 6: is a schematic block diagram of an ultrasound system in accordance with an embodiment of the present disclosure.
- FIG. 7: is a schematic block diagram of a 3D ultrasound image acquisition unit in the ultrasound system in accordance with an embodiment of the present disclosure.
- FIG. 8: is a schematic diagram showing a scan direction of 2D slice images of the 3D ultrasound image in accordance with an embodiment of the present disclosure.
- FIG. 9: is an illustrative embodiment showing volume data in accordance with the present disclosure.
- FIG. 10: is a side view of a 2D slice image selection unit in accordance with an embodiment of the present disclosure.
- FIG. 11: is an illustrative embodiment of the orientation and position transition of a control volume unit in the 2D slice image selection unit in accordance with the present disclosure.
- FIG. 12: is an illustrative embodiment of the transited coordinates system of the control volume unit in accordance with the present disclosure.
- FIG. 13: is a schematic block diagram of a processor in accordance with an embodiment of the present disclosure.
- FIG. 14: is a flow chart showing a process of selecting a 2D slice image from the 3D ultrasound image using the 2D slice image selection unit in accordance with one embodiment of the present disclosure.
- FIG. 15: is a diagram for illustrating a selection process of a 2D slice image from the 3D ultrasound image in accordance with another embodiment of the present disclosure.
- FIG. 16: is a flow chart showing a process of selecting a 2D slice image from the 3D ultrasound image using the 2D slice image selection unit in accordance with another embodiment of the present disclosure.

### DETAILED DESCRIPTION

A detailed description is provided with reference to the accompanying drawings. One of ordinary skill in the art can realize that the following description is illustrative only and is not in any way limiting. Other illustrative embodiments readily suggest themselves to such skilled persons having the benefit of this disclosure.

Referring to FIG. 6, there is shown a schematic block diagram of an ultrasound system 100 in accordance with an embodiment of the present disclosure. As shown in FIG. 6, the ultrasound system 100 comprises a three-dimensional (3D) ultrasound image acquisition unit 110, a 2D slice image selection unit 120, a processor 130 and a display unit 140.

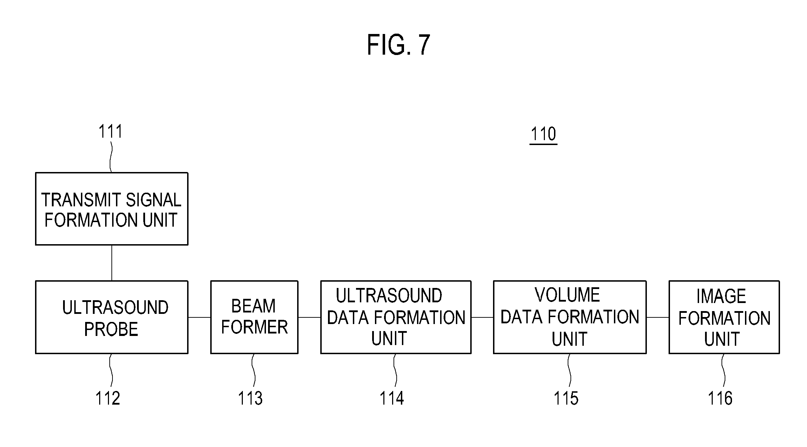

The 3D ultrasound image acquisition unit 110 is configured to transmit ultrasound signals to a target object and receive reflected ultrasound signals, i.e., ultrasound echo signals, from the target object to acquire ultrasound data thereof. The organization of the 3D ultrasound image acquisition unit 110 will be described later with reference to FIG. 7.

Referring to FIG. 7, there is shown a schematic block diagram of the 3D ultrasound image acquisition unit 110 in accordance with an embodiment of the present disclosure. As shown in FIG. 7, the 3D ultrasound image acquisition unit 110 comprises a transmit signal formation unit 111, an ultrasound probe 112 having a plurality of transducer elements (not shown), a beam former 113, an ultrasound data formation unit 114, a volume data formation unit 115 and an image formation unit 116.

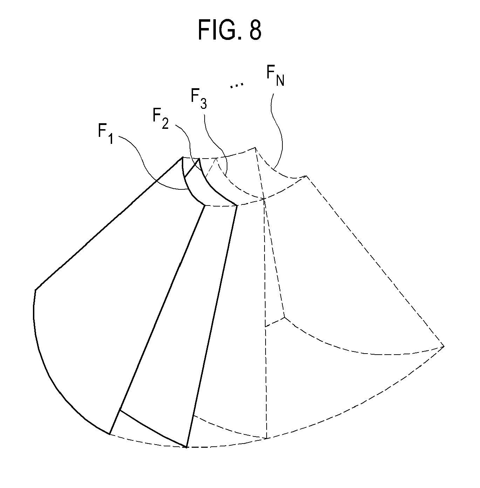

The transmit signal formation unit 111 is configured to form transmit signals in consideration of positions and focusing points of the transducer elements. The transmit signal formation unit 111 is configured to form the transmit signals sequentially and repeatedly. Thus, the transmit signal formation unit 111 is configured to form the transmit signals for obtaining image frames Fᵢ (1≤i≤N, N being an integer) as shown in FIG. 8. In FIG. 8, the image frames F; (1≤i≤N) are represented in the form of a fan-shaped image frame, although they are not limited thereto.

In response to the transmit signals from the transmit signal formation unit 111, the ultrasound probe 112 is configured to convert the transmit signals into corresponding ultrasound signals and transmit them to the target object. The ultrasound probe 112 is further configured to receive ultrasound echo signals reflected from the target object to form receive signals. The receive signals can be analog signals. The ultrasound probe 112 is configured to transmit the ultrasound signals and receive the ultrasound echo signals to form the receive signals in response to the transmit signals from the transmit signal formation unit 111. In an exemplary embodiment, the ultrasound probe 112 includes at least one of a 3D mechanical probe, a 2D array probe and the like.

In response to the receive signals from the ultrasound probe 112, the beam former 113 is configured to convert the receive signals from analog to digital to form digital signals corresponding thereto. The beam former 113 is further configured to receive-focus the digital signals in consideration of the positions and focusing points of the transducer elements in the ultrasound probe 112 to form a receive-focus beam.

The ultrasound data formation unit 114 is configured to form ultrasound data based on the receive-focus beam from the beam former 113. For example, the ultrasound data formation unit 114 is configured to form the ultrasound data corresponding to the respective frames Fᵢ (1≤i≤N) shown in FIG. 8 based on the receive-focus beam provided from the beam former 113.

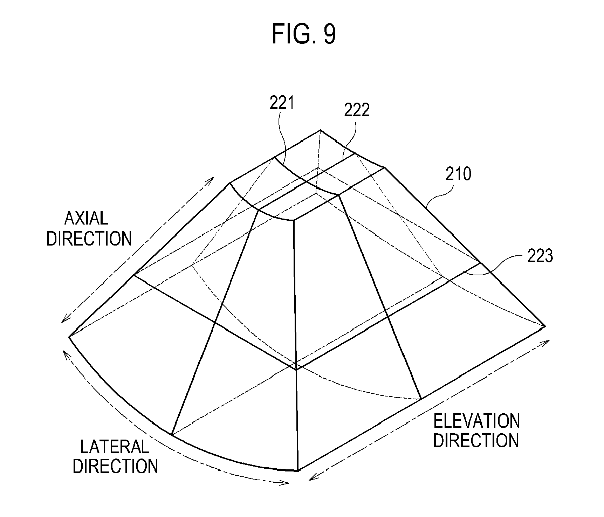

The volume data formation unit 115 is configured to form volume data 210 shown in FIG. 9 based on the ultrasound data from the ultrasound data formation unit 114. The volume data comprises the frames Fᵢ (1≤i≤N) and include a plurality of voxels with brightness values. In an exemplary embodiment, as shown in FIG. 9, reference numbers 221 to 223 indicate cross-sections, which are crossed at right angles. Also, as shown in FIG. 9, an axial direction indicates a propagation direction of the ultrasound signals starting from the transducer elements of the ultrasound probe 112, a lateral direction represents a scan line direction of the ultrasound signals, and an elevation direction depicts a depth direction of a 3D ultrasound image.

The image formation unit 116 is configured to render the volume data from the volume data formation unit 115 to form a 3D ultrasound image. In an exemplary embodiment, rendering of the image formation unit 116 includes ray-casting rendering, surface rendering and the like.

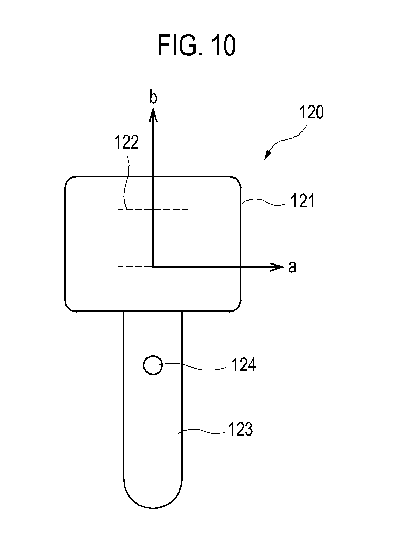

Referring back to FIG. 6, the 2D slice image selection unit 120 is configured to select cross-sections, i.e., 2D slice images, of the 3D ultrasound image from the 3D ultrasound image acquisition unit 110 by the processor 130. A detailed description of the 2D slice image selection unit 120 will be made with reference to FIG. 10.

Referring to FIG. 10, there is shown a side view of the 2D slice image selection unit 120. As shown in FIG. 10, the 2D slice image selection unit 120 includes a control volume unit 121, an orientation and position recognition unit 122, a grip 123 and an operation button 124, which is configured to receive input data from the operator for the operations of the 2D slice image selection unit.

The operation button 124 is provided on the grip 123 to receive input data from the operator. For example, the input data includes first and second input data, the first input data containing data for matching the coordinates of the control volume unit to that of the 3D ultrasound image and the second input data containing data for selecting a final 2D slice image.

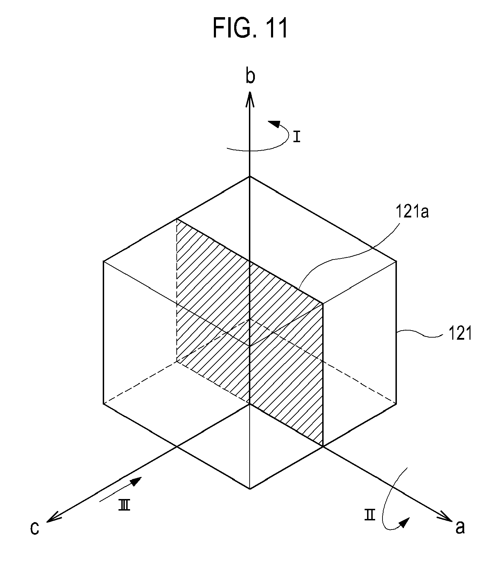

As shown in FIG. 11, the control volume unit 121 is positioned on the Cartesian coordinate system with an a-axis, a b-axis and a c-axis, which are crossed at right angles. In FIG. 11, a reference numeral 121a represents a selection plane to select a 2D slice image from the 3D ultrasound image, wherein at least one selection plane is formed by the control volume unit as a reference plane to select at least one 2D slice image from the 3D ultrasound image.

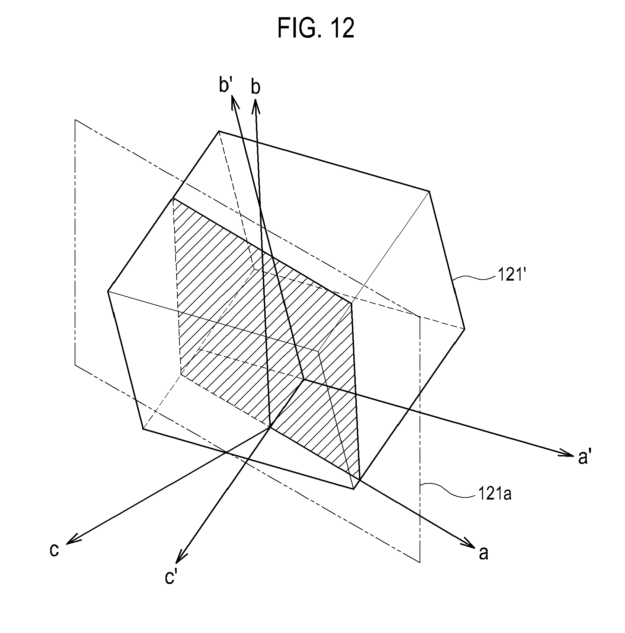

In case that the operator rotates the control volume unit 121 centering on the b-axis (represented in FIG. 11 as an arrow I) and centering on the a-axis (represented in FIG. 11 as an arrow II), and/or moves it toward a direction of the c-axis (represented in FIG. 11 as an arrow III), the orientation and position of the control volume unit 121 is transited to that of a control volume unit 121' as shown in FIG. 12. Since the orientation and position of the selection plane 121 a is fixed on the Cartesian coordinate system, the relative coordinate system of the selection plane 121a to the 3D ultrasound image 121 can be changed according to the operator's operation.

In an exemplary embodiment, the control volume unit 121 has the shape of, for example, a rectangular solid. In another embodiment, the control volume unit 121 has a fan shape similar to a shape of the 3D ultrasound image, which has curved top and bottom portions. With the fan shape, the operator can intuitively match the 3D ultrasound image to the control volume unit 121.

The orientation and position recognition unit 122 is mounted within the control volume unit 121 to recognize the orientation and position thereof. In an exemplary embodiment, the processor 130 is connected to the 3D ultrasound image acquisition unit 110 and the 2D slice image selection unit 120, and the orientation and position recognition unit 122 includes a sensor (not shown) configured to detect the rotation and/or movement of the control volume unit 121 to form detection signals corresponding to the rotation and/or movement thereof. The processor 130 forms orientation and position signals for determining the orientation and/or position of the control volume unit 121 based on the detection signals from the sensor. The sensor comprises a device that detects the rotation and/or movement of the control volume unit 121. For example, the sensor includes an inertial sensor, a gyro sensor, an acceleration sensor and the like. Further, the processor 130 includes a micro controller unit (MCU) to form the orientation and position signals for determining the orientation and/or position of the control volume unit 121 based on the detection signals from the sensor.

The grip 123 has a shape of a stick that is projected from one surface of the control volume unit 121. Such a shape allows the operator to seize the grip 123 and change the orientation and/or position of the control volume unit 121 of 2D slice image selection unit 120.

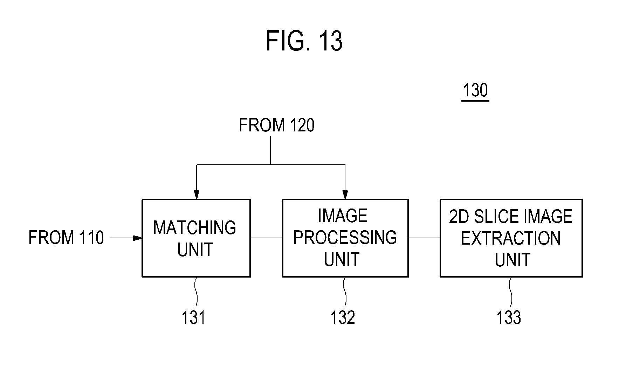

Referring back to FIG. 6, in an exemplary embodiment, the processor 130 is connected to the 2D slice image selection unit 120 through wireless communication channels. The processor 130 is configured to match the coordinate system of the 3D ultrasound image from the 3D ultrasound image acquisition unit 110 to that of the control volume unit 121. The processor 130 is configured to extract a slice corresponding to the selection plane 121a from the 3D ultrasound image. A detailed description of the processor 130 will be made with reference to FIG. 13.

Referring to FIG. 13, there is shown a schematic block diagram of the processor 130 in accordance with an embodiment of the present disclosure. As shown in FIG. 13, the processor 130 includes a matching unit 131, an image processing unit 132 and a 2D slice image extraction unit 133. The matching unit 131 is configured to match the coordinate system of the control volume unit 121 to that of the 3D ultrasound image in response to first input data from the 2D slice image selection unit 120.

In response to an orientation and position signal from the 2D slice image selection unit 120, the image processing unit 132 is configured to transit the orientation and position of the 3D ultrasound image (i.e., moving and rotating of the 3D ultrasound image) to correspond them to the orientation and position of the control volume unit 121, which are changed according to the orientation and position signal. For example, as shown in FIG. 12, the image processing unit 132 matches the a'-, b'- and c'-axes of the control volume unit 121' to the X-, Y- and Z-axes of the 3D ultrasound image 10 of FIG. 4, in response to the orientation and position signal. This is so that the orientation and position of the 3D ultrasound image is transited. As such, the coordinate system of the selection plane 121a corresponds to the coordinate system of the slice of the 2D slice image 11' to be displayed, as shown in FIG. 4. Thus, the operator selects any one 2D slice image from the 3D ultrasound image by rotating and moving the control volume unit 121.

The operation button 124 is provided on the grip 123 to activate operations. In an exemplary embodiment, the operations include a first operation defined as the first input data and a second operation defined as the second input data. For example, when the operation button 124 is activated in response to the first operation (i.e., the first input data is provided), the matching unit 131 matches the coordinate system of the control volume unit 121 to that of the 3D ultrasound image 10. When the operation button 124 is activated in response to the second operation (i.e., the second input data is provided), the 2D slice image extraction unit 133 extracts a 2D slice image on a slice corresponding to the fixed selection plane 121 a from the 3D ultrasound image 10.

Referring back to FIG. 6, the display unit 140 is configured to display the 3D ultrasound image from the 3D ultrasound image acquisition unit 110. Further, the display unit 140 is configured to display the selected 2D slice image from the 3D ultrasound image.

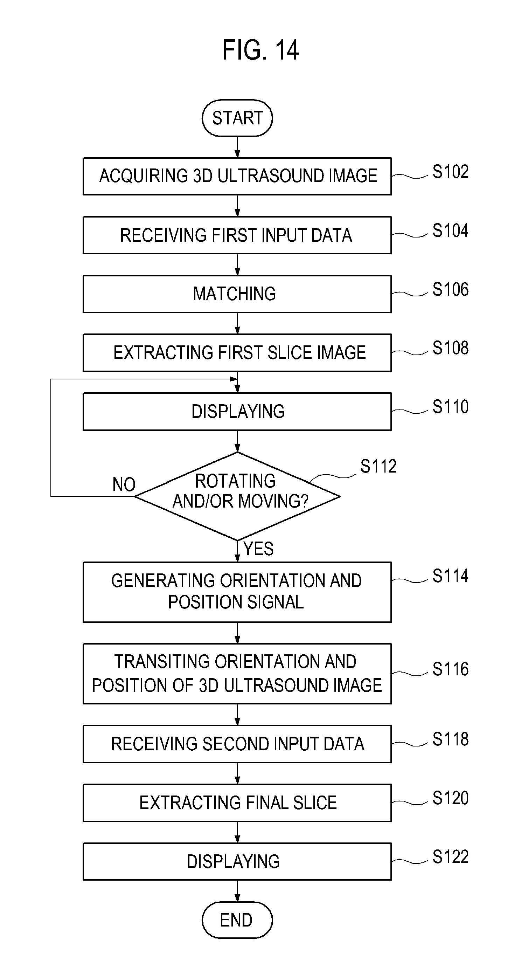

Referring to FIG. 14, there is shown a flow chart illustrating a process of selecting the 2D slice image from the 3D ultrasound image using the 2D slice image selection unit in accordance with an embodiment of the present disclosure. As shown in FIG. 14, the 3D ultrasound image acquisition unit 110 transmit ultrasound signals to a target object and receive ultrasound echo signals reflected therefrom to acquire a 3D ultrasound image of the target object (S102).

When a first operation of the operation button 124 is activated (i.e., the first input data is provided from the operator) (S104), the matching unit 131 of the processor 130 matches the coordinate system of the control volume unit 121 to that of the 3D ultrasound image (S106). The 2D slice image extraction unit 133 extracts a first 2D slice image corresponding to the selection plane 121a from the 3D ultrasound image of which the coordinate system is matched to that of the control volume unit 121 (S108). The display unit 140 displays the matched 3D ultrasound image and the first 2D slice image (S110).

The processor 130 decides whether or not the operator rotates and/or moves the control volume unit 121 due to the detection signals from the orientation and position recognition unit 122 (S 112). If it is determined that the control volume unit 121 is moved and/or rotated, then the processor 130 forms an orientation and position signal, which represents the amount of rotation and/or movement of the control volume unit 121 (S114). Otherwise, if it is determined that the control volume unit 121 is not moved and/or rotated, then the processor 130 do not form the orientation and position signal.

In response to the orientation and position signal, the image processing unit 132 transits the orientation and position of the 3D ultrasound image to the changed orientation and position of the control volume unit 121 (S116).

When a second operation of the operation button 124 is activated (i.e., the second input data is provided from the operator) (S118), the 2D slice image extraction unit 133 extracts a final 2D slice image corresponding to the selection plane 121a from the transited 3D ultrasound image (S120). Thereafter, the display unit 140 displays the final 2D slice image from the transited 3D ultrasound image (S122).

Although the matching the control volume unit 121 to the 3D ultrasound image 10 is described in the exemplary embodiment, it is possible that the coordinate system of the 3D ultrasound image 10 shown in FIG. 4 is fixed and the changed coordinate system of the control volume unit 121, i.e., a'-, b'- and c'-axes, can be matched to the coordinate system of the selected plane corresponding to the slice of the 2D slice image 11' to be displayed, i.e., X'-, Y'- and Z'-axes.

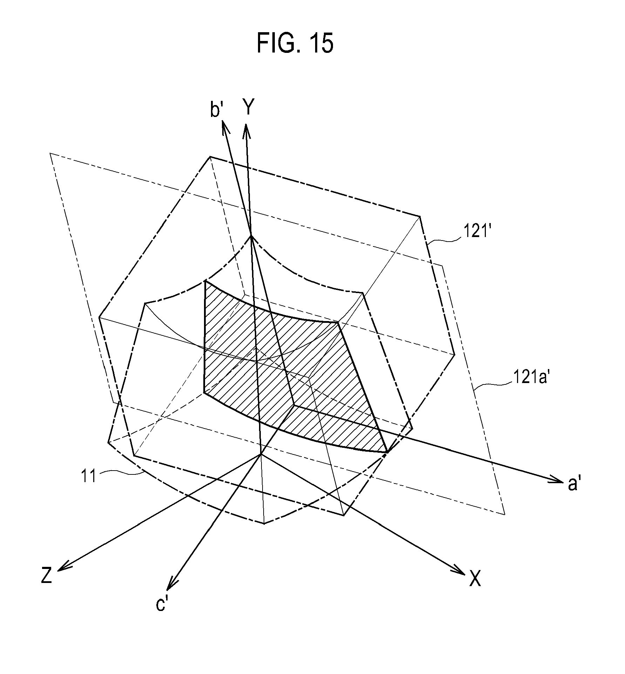

FIG. 15 is a schematic view showing a slice selecting process to acquire a 2D slice image at an arbitrary slice according to another embodiment of the present disclosure. In the previous embodiment, the orientation and position of the control volume unit 121 and those of the 3D ultrasound image 10 are changed relative to the selection plane 121a. However, in this embodiment, the coordinate system of the 3D ultrasound image 10 with X-axis, Y-axis and Z-axis is fixed, while the control volume unit 121' and selection plane 121a' can be moved or rotated relative to the 3D ultrasound image with coordinates a'-axis, b'-axis and c'-axis, which represents the changed orientation and position of the control volume unit 121, as shown in FIG. 15. In this embodiment, the elements such as the matching unit can be removed since the selection plane moves and rotates with the control volume unit together. Further, the 3D ultrasound image is fixed and a 2D slice image on a slice corresponding to the selection plane 121a' can be extracted therefrom.

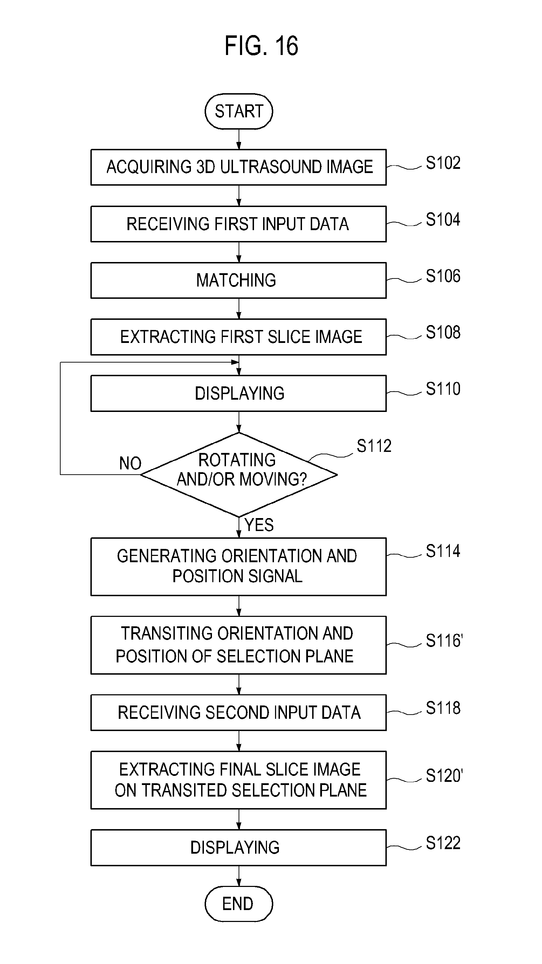

In this embodiment, in response to the orientation and position signal, the processor 130 transits the orientation and position of the selection plane 121 a' as shown in FIG. 15. Thus, the process to selecting a 2D slice image from the 3D ultrasound image is slightly changed. As shown in FIG. 16, in response to the orientation and position signal, the image processing unit 132 transits the orientation and position of the selection plane 121a to the changed orientation and position of the control volume unit 121 (S116'). Further, when a second operation of the operation button 124 is activated (i.e., the second input data is provided from the operator) (S118), the 2D slice image extraction unit 133 extracts a final 2D slice image on a slice corresponding to the transited selection plane 120a' from the 3D ultrasound image (S120').

According to the embodiments described above, there is a selection plane to select a 2D slice image. However, there is a plurality of selection planes to select a plurality 2D slice images at one time.

According to the present disclosure, the operator can change the orientation and position of the 3D ultrasound image by changing the orientation or position of the control volume unit. Thus, the operator can easily select a slice to be displayed by using the control volume unit without performing any complex button operation.

Although embodiments have been described with reference to a number of illustrative embodiments thereof, it should be understood that various other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a 3D ultrasound image acquisition unit configured to acquire a 3D ultrasound image of a target object;
a 2D slice image selection unit including a control volume unit, the 2D slice image selection unit being configured to be rotated and/or moved by an operator, wherein at least one selection plane is formed by the control volume unit as a reference plane for selecting at least one 2D slice image from the 3D ultrasound image; and
a processor coupled to the 3D ultrasound image acquisition unit and the 2D slice image selection unit, the processor being configured to extract at least one 2D slice image corresponding to the at least one selection plane from the 3D ultrasound image;
wherein the 3D ultrasound image or the at least one selection plane is rotated and/or moved together with the control volume unit.

2. The ultrasound system of Claim 1, wherein the at least one selection plane is fixed while the control volume unit and the 3D ultrasound image are rotated and/or moved together, and wherein the processor matches a coordinate system of the control volume unit to a coordinate system of the 3D ultrasound image to rotate and/or move the 3D ultrasound image and the control volume unit together relative to the at least one selection plane.

3. The ultrasound system of Claim 2, wherein a shape of the control volume unit corresponds to a shape of the 3D ultrasound image.

4. The ultrasound system of Claim 2, wherein the 2D slice image selection unit includes:
an orientation and position recognition unit mounted on the control volume unit,
the orientation and position recognition unit being configured to detect the rotation and/or movement of the control volume unit to form an orientation and
position signal of the control volume unit;
a grip coupled to the control volume unit; and
an operation button formed on the grip, the operation button being configured to receive input data for operations of the 2D slice image selection unit from the operator.

5. The ultrasound system of Claim 4, wherein the orientation and position recognition unit includes a sensor configured to detect the rotation and/or movement of the control volume unit to form detection signals, and
wherein the processor is further configured to generate the orientation and position signal of the control volume unit based on the detection signals.

6. The ultrasound system of Claim 5, wherein the processor includes:
a matching unit configured to match the coordinate system of the control volume unit to the coordinate system of the 3D ultrasound image;
an image processing unit configured to change the orientation and position of the 3D ultrasound image corresponding to the changed orientation and position of the control volume unit based on the orientation and position signal; and
a 2D slice image extraction unit configured to extract the at least one 2D slice image corresponding to the at least one selection plane from the 3D ultrasound image.

7. The ultrasound system of Claim 6, wherein if the operation button receives input data of a first operation, then the matching unit matches the coordinate system of the control volume unit to the coordinate system of the 3D ultrasound image, and
if the operation button receives input data of a second operation, then the 2D slice image extraction unit extracts the at least one 2D slice image corresponding to the at least one selection plane from the 3D ultrasound image.

8. The ultrasound system of Claim 1, wherein the 3D ultrasound image is fixed while the control volume unit and the at least one selection plane are rotated and/or moved together, and wherein the processor matches a coordinate system of the control volume unit to a coordinate system of the at least one selection plane to rotate and/or move the at least one selection plane and the control volume unit together relative to the 3D ultrasound image.

9. The ultrasound system of Claim 8, wherein the 2D slice image selection unit includes:
an orientation and position recognition unit mounted on the control volume unit,
the orientation and position recognition unit being configured to detect the rotation and/or movement of the control volume unit to form an orientation and
position signal of the control volume unit;
a grip coupled to the control volume unit; and
an operation button formed on the grip, the operation button being configured to receive input data for operations of the 2D slice image selection unit from the operator.

10. The ultrasound system of Claim 9, wherein the orientation and position recognition unit includes a sensor configured to detect the rotation and/or movement of the control volume unit to form detection signals, and
wherein the processor is further configured to generate the orientation and position signal of the control volume unit based on the detection signals.

11. The ultrasound system of Claim 10, wherein the processor includes:
a matching unit configured to match the coordinate system of the control volume unit to the coordinate system of the at least one selection plane;
an image processing unit configured to change the orientation and position of the at least one selection plane corresponding to the changed orientation and
position of the control volume unit based on the orientation and position signal;
and
a 2D slice image extraction unit configured to extract the at least one 2D slice image corresponding to the at least one selection plane from the 3D ultrasound image.

12. The ultrasound system of Claim 11, wherein if the operation button receives input data of a first operation, then the matching unit matches the coordinate system of the control volume unit to the coordinate system of the at least one selection plane, and
if the operation button receives input data of a second operation, then the 2D slice image extraction unit extracts the at least one 2D slice image corresponding to the at least one selection plane from the 3D ultrasound image.

13. A method of selecting at least one 2D slice image from a 3D ultrasound image, comprising:
a) acquiring a 3D ultrasound image of a target object;
b) matching a coordinate system of the 3D ultrasound image to a coordinate system of a control volume unit configured to be moved and/or rotated by an operator, wherein at least one selection plane is formed by the control volume unit as a reference plane for selecting at least one 2D slice image from the 3D ultrasound image;
c) detecting an orientation and position of the control volume unit;
d) rotating and/or moving the 3D ultrasound image or the at least one selection plane together with the control volume unit; and
e) extracting at least one 2D slice image corresponding to the at least one selection plane from the 3D ultrasound image.

14. The method of Claim 13, wherein the at least one selection plane is fixed while the control volume unit and the 3D ultrasound image are rotated and/or moved together.

15. The method of Claim 13, wherein the 3D ultrasound image is fixed while the control volume unit and the at least one selection plane are rotated and/or moved together.
